# DEMANDE DE BREVET EUROPEEN

(11) **EP 3 593 789 A1**
(43) Date de publication de la demande: **15.01.2020**
(21) Numéro de dépôt: 18305933.6
(22) Date de dépôt: 11.07.2018
(51) Int. Cl.: A61K 9/08, A61K 47/44, A61K 31/00

(54) **COMPOSITION CONTENANT UN DÉRIVÉ DE 7BETA-HYDROXYCHOLESTÉROL ET UN VÉHICULE LIPIDIQUE, ET SON UTILISATION DANS LE TRAITEMENT DE PATHOLOGIES NÉOPLASIQUES**

(71) Demandeur: Beta Innov, 75015 Paris (FR)
(72) Inventeur: MERSEL, Marcel, 34090 MONTPELLIER (FR); RAKOTOARIVELO, Clovis, 34070 MONTPELLIER (FR)
(74) Mandataire: Santarelli

(57) **Abrégé**

L'invention concerne une composition comprenant un dérivé de 7beta-hydroxycholestérol et un véhicule lipidique, notamment une huile végétale, et son utilisation dans le traitement de pathologies néoplasiques, telles que le glioblastome multiple. Ladite composition peut être administrée par voie orale.

## Description

L'invention concerne une composition comprenant un dérivé de 7beta-hydroxycholestérol et un véhicule lipidique, son procédé de préparation et son utilisation dans le traitement de pathologies néoplasiques, en particulier le glioblastome multiple.

En particulier, l'invention concerne une composition comprenant un dérivé de 7beta-hydroxycholestérol et un véhicule lipidique, à l'exclusion d'un véhicule lipidique consistant en des vésicules formées d'une ou plusieurs couche(s) lipidique(s), telles que, par exemple, les vésicules de type liposomes ou micelles.

Avantageusement, ladite composition est sous forme liquide et peut être administrée par voie orale.

En particulier, ledit dérivé de 7beta-hydroxycholestérol est en solution dans ledit véhicule lipidique.

Le glioblastome multiple (GBM), ou astrocytome de grade 4, est une tumeur cérébrale caractérisée essentiellement par la transformation de la cellule gliale, dont fait partie la cellule astrocytaire, en cellule cancéreuse.

Malgré les avancées scientifiques et thérapeutiques substantielles en cancérologie, comme dans les domaines de l'immunothérapie et des nanotechnologies, le GBM reste un cancer incurable. Au mieux, chercheurs et médecins sont satisfaits lorsque la médiane de vie des malades peut être prolongée au maximum de quinze mois.

Des problèmes majeurs posés par le traitement du GBM sont : (a) la rechute causée par les cellules souches (en anglais « stem cells »). En effet, lorsque les thérapies existantes parviennent à éradiquer tout ou partie de la tumeur, les cellules souches sont souvent à l'origine d'un nouveau départ tumoral; (b) les molécules utilisées en chimiothérapie traversent difficilement la Barrière Hémato-Tumorale (BHT) et de ce fait de faibles quantités de l'agent anti-GBM atteignent le site tumoral. L'utilisation de mini-pompes (Alzet®), de réservoirs (Mamiya®) ou de supports (Gliadel®) implantés dans la tumeur GBM afin de contourner le problème posé par la BHT n'améliore pas significativement l'efficacité de la chimiothérapie. L'effet d'une irradiation du cerveau par des ultrasons, pour assouplir la BHT, sur l'efficacité de la chimiothérapie est actuellement en étude chez l'animal.

En chimiothérapie, un des traitements « leader » est une bithérapie qui consiste à administrer de l'avastin® (inhibition de l'association du VEGF à ses récepteurs) et de l'irinotecan® (inhibiteur de la topoisomérase I). La trithérapie de type PVC (Procarbazine, agent alkylant d'ADN ; Vincristine, inhibition de la polymérisation des microtubules ; CCNU, agent alkylant non spécifique) est à l'heure actuelle très controversée. Le temozolomide, agent alkylant de la guanine, en association avec une radiothérapie, montre une augmentation de la médiane de vie de 2 à 3 mois pour les patients qui ont un ADN hyperméthylé. (protocole Stupp). Des essais cliniques utilisant l'approche TumorTreating Fields (TTF) comme supplément au protocole Stupp sont en cours. Des essais cliniques (phase III) qui testent le cilengitide (inhibition de quelques récepteurs à l'intégrine) et le talampanel (blocage des canaux au glutamate de type AMPA) sont en cours.

Aussi, des chimiothérapies montrant une efficacité pour des cancers autres que le GBM n'améliorent pas la médiane de vie pour les patients souffrant de GBM. Citons par exemple, la bléomycine (administrée pour le lymphome de Hodgkin), l'afatinib dimaléate et le cisplatine (administrés pour le cancer du poumon non à petites cellules) et le cyclophosphamide (administré pour le cancer du sein).

La demande WO2013/168096 décrit une famille de dérivés de 7beta-hydroxycholestérol montrant une efficacité anti tumorale sur des cellules cancéreuses humaines in vitro , dont le GBM, notamment sous forme liposomale ou sous forme de solution éthanolique. En particulier, le composé préparé dans l'exemple 6 de cette demande (dénommé dans les exemples de la présente demande « composé BIM2b ») a montré une activité in vitro sur une lignée de GBM humaine, la lignée U87-MG (U 87).

Il existe donc un besoin de disposer d'une formulation permettant audit dérivé de 7beta-hydroxycholestérol, à savoir une molécule ayant une activité thérapeutique, de traverser l'obstacle de la Barrière Hémato-Tumorale (BHT), afin d'atteindre, en particulier, une tumeur localisée dans le cerveau, tout en contenant une quantité suffisante de cette molécule thérapeutique.

Il est également recherché une formulation permettant une administration aisée, de préférence par voie orale, et qui n'entraine pas d'effets toxiques sur une durée de traitement pouvant atteindre plusieurs semaines, voire plusieurs mois, en tenant compte de l'état de santé précaire des patients.

Les composés stéroïdiens, comme les hormones stéroïdes, les minéralocorticoïdes et les glucocorticoïdes sont dépourvus de la chaîne latérale qui, elle, fait partie du cholestérol et de la famille des oxystérols. Aussi, dans certains composés stéroïdiens, on trouve des fonctions cétones combinées à des fonctions alcools et des noyaux aromatiques, ce qui n'est pas le cas pour le cholestérol et les oxystérols les plus communs.

De ce fait, les composés stéroïdiens se dissolvent habituellement plus facilement dans des huiles que le cholestérol et les dérivés du 7beta-hydroxycholestérol, en particulier les composés de formule (I) ci-dessous, pour les raisons suivantes :
- l'encombrement stérique des composés stéroïdiens est moindre que celui du cholestérol et les dérivés du 7beta-hydroxycholestérol mentionnés ci-dessus,
- les composés stéroïdiens possèdent plus de fonctions chimiques et de cycles possédant des électrons délocalisés que le cholestérol et les dérivés du 7beta-hydroxycholestérol, et
- les fonctions chimiques et structures cycliques à caractères polaires retrouvées dans les composés stéroïdiens permettent une association aux fonctions acides, alcools, doubles liaisons et certains cycles (spinastérol, schotténol) retrouvé dans l'huile et, donc, une meilleure dissolution dans l'huile que le cholestérol et les dérivés du 7beta-hydroxycholestérol de formule (I) ci-dessous.

On a maintenant trouvé qu'une formulation associant un dérivé de 7beta-hydroxycholestérol et un véhicule lipidique, à l'exclusion d'un véhicule lipidique consistant en des vésicules lipidiques formées d'une ou plusieurs couche(s) lipidique(s), permettait le passage de la BHT de ce dérivé de stérol ainsi que l'obtention d'une activité anti-GBM in vivo remarquable chez l'animal. Avantageusement, ledit dérivé de 7beta-hydroxycholestérol est en solution dans ledit véhicule lipidique.

Ledit véhicule lipidique est, en particulier, un véhicule lipidique acceptable sur le plan pharmaceutique.

Par « vésicule lipidique formée d'une ou plusieurs couche(s) lipidique(s) », on entend une vésicule lipidique comprenant ou constituée d'une ou plusieurs couche(s) lipidique(s), telles que, par exemple, les vésicules de type liposomes ou micelles.

Avantageusement, on a également trouvé que cette formulation est stable à un pH d'environ 1,5 (pH de la digestion gastrique chez l'homme) et à un pH d'environ 8,5 à 10 (pH de la digestion au niveau de la zone de Vater chez l'homme), ce qui est particulièrement recherché pour permettre au composé actif d'atteindre le site d'action, dans le cadre d'une administration par voie orale.

L'invention concerne donc, selon un premier aspect, une composition comprenant au moins un dérivé de 7beta-hydroxycholestérol et un véhicule lipidique, à l'exclusion d'un véhicule lipidique consistant en des vésicules lipidiques formées d'une ou plusieurs couche(s) lipidique(s), dans laquelle ledit dérivé de 7beta-hydroxycholestérol répond à la formule (I) 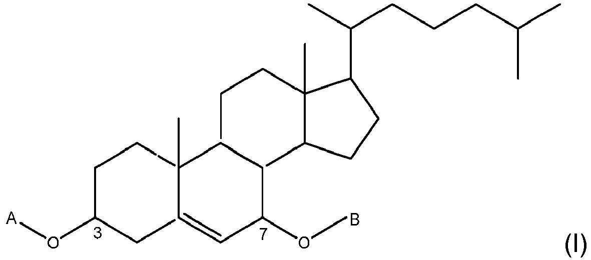 dans laquelle :
- A représente
   un groupement -C(O)R₁ dans lequel R₁ est un hétérocycle saturé comprenant 5 à 14 membres et incluant 1 ou 2 hétéroatomes, non substitué ou substitué par au moins un alkyle en C₁-C₆ linéaire ou ramifié ou un groupement choisi parmi OR, NRR', NHR et SR, où est R et R', indépendamment, représentent l'hydrogène, un alkyle en C₁-C₁₂, de préférence en C₁-C₆, linéaire ou ramifié, ou un aryle non substitué ;
   ou un groupement -(R₂)ₙ- dans lequel R₂ est un reste d'acide aminé lié par son extrémité C-terminale et n= 1 à 3, chacun des R₂ étant identique ou différent, dans lequel l'extrémité N-terminale dudit acide aminé peut être substituée par un groupe -C(O)R₃ dans lequel R₃ est un groupe arylalkyl mono-ou pluri-cyclique en C₆-C₁₄ ; un groupe hétéroarylalkyl mono-ou pluri-cyclique en C₅-C₁₄ pouvant comporter un ou plusieurs hérétoatomes, identiques ou différents; un groupe arylalkyloxy mono-ou pluri-cyclique en C₆-C₁₄ ou un groupe hétéroarylalkyloxy mono-ou pluri-cyclique en C₅-C₁₄ pouvant comporter un ou plusieurs hérétoatomes, identiques ou différents,
- B représente un groupement -C(O)R₄, dans lequel R₄ est un alkyle en C₁-C₁₂, de préférence en C₁-C₆, linéaire ou ramifié, non substitué ou substitué par un groupement choisi parmi OR, NRR', NHR et SR, tels que définis ci-dessus; un groupe aryle, non substitué ou substitué par un groupement choisi parmi OR, NRR', NHR et SR, tels que définis ci-dessus; ou encore R₄ représente OR₅, dans lequel R₅ est un alkyle en C₁-C₁₂, de préférence en C₁-C₆, linéaire ou ramifié.

Le groupement alkyle désigne un groupement linéaire ou ramifié en C₁-C₁₂ tel que les groupements méthyle, éthyle, propyle, isopropyle, butyle, isobutyle, sec-butyle, tert-butyle, pentyle, isopentyle, sec-pentyle, tert-pentyle, néo-pentyle, hexyle, isohexyle, sec-hexyle, tert-hexyle, heptyle, octyle, nonyle, décyle, undécyle ou dodécyle, les groupements alkyles linéaires ou ramifiés en C₁-C₆ étant préférés.

Le groupement aryle désigne un groupement insaturé, monocyclique ou pluricyclique, carbocyclique, en C₆-C₁₄, tel que les groupements phényle, naphtyle, indényle, anthracényle et plus particulièrement le groupement phényle.

Par « hétérotaome », on entend un atome d'oxygène, d'azote ou de soufre.

Des restes d'acides aminés avantageux sont, par exemple, des motifs méthionyl, glycinyl ou alanyl.

La préparation des composés de formule (I) est décrite dans les demandes EP2666382 et WO2013/168096.

Par « comprenant », on entend, au sens de la présente description, aussi bien « contenant » que « constitué(e) de ».

De préférence, le véhicule lipidique comprend une huile ou un mélange d'huiles, avantageusement une huile végétale ou un mélange d'huiles végétales.

En particulier, ledit véhicule lipidique est constitué d'une huile ou d'un mélange d'huiles, avantageusement d'une huile végétale ou d'un mélange d'huiles végétales.

On entend par « huile végétale » un corps gras extrait d'une plante oléagineuse, c'est-à-dire une plante dont certaines parties, telles que les graines, noix ou fruits contiennent des lipides, en particulier une huile comestible.

Ladite huile végétale peut être choisie, par exemple, parmi l'huile d'argan, l'huile d'avocat, l'huile de lin, l'huile de tournesol, de palme, de palmiste, de noix de coco, de pépins de raisins, de moutarde noire, d'oeillette, de beurre de karité, d'amande douce, de soja, d'arachide, de coton, de sésame, d'olive, de maïs, de cacao, de ricin, de Moringa (ou huile de Ben), de colza, de rocouyer, de germe de blé, de carthame, de noix, de noisette, de navette ou leurs mélanges.

On utilisera, de préférence, une huile végétale à faible teneur en vitamine E, en particulier l'huile d'argan.

L'utilisation d'une huile ou d'un mélange d'huiles, avantageusement une huile végétale ou un mélange d'huiles végétales, en tant que véhicule lipidique dans la composition, permet d'obtenir une composition pouvant contenir une quantité de composé de formule (I) suffisante pour obtenir à la fois le passage de la Barrière Hémato-Tumorale (BHT) et l'activité thérapeutique recherchée.

Lesdites huiles, en particulier les huiles végétales, sont, de préférence, mises en oeuvre sous une forme pharmaceutiquement acceptable.

De plus, les formulations selon l'invention comprenant un tel véhicule lipidique présentent une très bonne stabilité à des pH extrêmes tels qu'un pH d'environ 1,5 (pH de la digestion gastrique chez l'homme) et à un pH d'environ 8,5 à 10 (pH de la digestion au niveau de la zone de Vater chez l'homme), ce qui permet de préserver l'activité du composé de formule (I). De telles propriétés n'ont pas été montrées dans le cas de vésicules lipidiques formées d'une ou plusieurs couche(s) lipidique(s), telles que, par exemple, les vésicules de type liposomes ou micelles.

Des composés préférés de formule (I) sont ceux dans laquelle:
- A représente un groupement -C(O)R₁ dans lequel R₁ est un hétérocycle saturé comprenant 5 à 14 membres et incluant 1 ou 2 hétéroatomes, non substitué ou substitué par au moins un alkyle en C₁-C₆ linéaire ou ramifié ;
- B représente un groupement -C(O)R₄, dans lequel R₄ est un alkyle en C₁-C₁₂, de préférence en C₁-C₆, linéaire ou ramifié ou R₄ représente OR₅, dans lequel R₅ est un alkyle en C₁-C₁₂, de préférence en C₁-C₆, linéaire ou ramifié.

Selon un aspect préféré, R₁ est un hétérocycle saturé comprenant 5 membres et incluant 2 atomes d'oxygène, substitué par au moins un alkyle en C₁-C₆ linéaire ou ramifié, notamment un groupe méthyle. En particulier R₁ est un groupe 2,2 diméthyl-1,3-dioxolane.

De préférence, R₄ est un alkyle en C₁-C₆, linéaire ou ramifié, en particulier un groupe méthyle.

Selon un aspect préféré, B représente un groupement acyl dans lequel le groupe alkyl est en C₁-C₆, en particulier acétyl ou encore un groupement alcoxycarbonyl dans lequel le groupe alkyl est en C₁-C₆, en particulier un groupement *tert*-butoxycarbonyl.

D'autres composés préférés de formule (I) sont ceux dans laquelle :
- A représente un groupement -(R₂)ₙ- dans lequel R₂ est un reste d'acide aminé et n = 2 ; ou
- A représente un groupement -(R₂)ₙ- dans lequel R₂ est un reste d'acide aminé, n = 2 et l'extrémité N-terminale dudit acide aminé est substituée par un groupe arylalcoxycarbonyl, en particulier benzyloxycarbonyl ; ou
- A représente un radical alanyl lié à un radical glycinyl , éventuellement substitué sur son extrémité N-terminale par un groupe arylalcoxycarbonyl, en particulier benzyloxycarbonyl; ou
- A représente un radical méthionyl lié à un radical glycinyl, éventuellement substitué sur son extrémité N-terminale par un groupe arylalcoxycarbonyl, en particulier benzyloxycarbonyl, et
- B est tel que défini ci-dessus, dans ses définitions générales ou préférées.

Des composés préférés de formule (I) sont les suivants :
- le 7-((*tert*-butoxycarbonyl)oxy)-10,13-diméthyl-17-(6-méthylheptan-2-yl)-2,3,4,7,8,9,10,11,12,13,14,15,16,17-tétradécahydro-1*H-*cyclopenta[a]phenanthren-3-yl 2-(2-(((benzyloxy)carbonyl)amino)-acétamido)propanoate, également dénommé par le nom simplifié « 3-benzyloxycarbonyl-glycinyl-alanyl-7-β-O-tert-butyloxycarbonyl-cholestérol » ou composé BIM1a ;
- le 7-acétoxy-10,13-diméthyl-17-(6-méthylheptan-2-yl)-2,3,4,7,8,9,10,11,12,13,14,15,16,17-tétradécahydro-1H-cyclopenta[a]phenanthren-3-yl 2-(2-(((benzyloxy)carbonyl)amino)-acétamido)propanoate, également dénommé par le nom simplifié « 3-benzyloxycarbonyl-glycinyl-alanyl-7-β-O-acétyl-cholestérol » ou composé BIM1b ;
- le 7-((tert-butoxycarbonyl)oxy)-10,13-diméthyl-17-(6-méthylheptan-2-yl)-2,3,4,7,8,9,10,11,12,13,14,15,16,17-tétradécahydro-1H-cyclopenta[a]phenanthren-3-yl 2,2-diméthyl-1,3-dioxolane-4-carboxylate, également dénommé par le nom simplifié « 3-(*S*)-2,2-diméthyl-1,3-dioxolane-4-carboxyl-7-β-O-tert-butyloxycarbonyl- cholestérol » ou composé BIM2a ;
- le 7-acétoxy-10,13-diméthyl-17-(6-méthylheptan-2-yl)-2,3,4,7,8,9,10,11,12,13,14,15,16,17-tétradécahydro-1H-cyclopenta[a]phenanthren-3-yl 2,2-diméthyl-1,3-dioxolane-4-carboxylate également dénommé par le nom simplifié « 3-(S)-2,2-diméthyl-1,3-dioxolane-4-carboxyl-7-β-O-acétyl-cholestérol », ou encore « cholest-5-ene-3,7-diol, 7-acetate 3-[[(4S)-2,2-dimethyl-1,3-dioxolan-4-yl]carboxylate], (3β,7β) » ou composé BIM2b.

Ces composés de formule (I) sont décrits, respectivement, dans les exemples 3, 4, 5 et 6 de la demande WO2013/168096.

Un composé de formule (I) particulièrement préféré aux fins de l'invention est le « composé BIM2b » mentionné ci-dessus, c'est-à-dire un composé de formule (I) dans lequel A est un groupement -C(O)R₁ dans lequel R₁ est un groupe 2,2-diméthyl-1,3-dioxolane et B est un groupement acétyl.

Ce composé répond à la formule

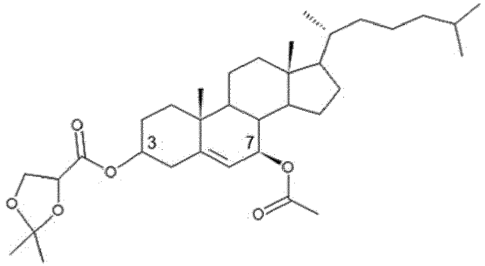

Le composé BIM1a est un composé de formule (I) dans laquelle A est un groupement 3-benzyloxycarbonyl-glycinyl-alanyl et B est un groupe tert-butoxycarbonyl.

Le composé BIM1b est un composé de formule (I) dans laquelle A est un groupement 3-benzyloxycarbonyl-glycinyl-alanyl et B est un groupe acétyl.

Le composé BIM2a un composé de formule (I) dans laquelle A est un groupement -C(O)R₁ dans lequel R₁ est un groupe 2,2-diméthyl-1,3-dioxolane et B est un groupe tert-butoxycarbonyl.

Dans la présente description, et sauf stipulation contraire, les gammes de valeurs indiquées s'entendent bornes incluses.

La teneur en composé de formule (I) dans la composition selon l'invention est, de préférence, de 0,1 à 3,5% (p/v) du volume total de la composition, de préférence 3%. De préférence, ladite composition comprend le composé de formule (I) à raison de 1 à 35 mg/ml, de préférence 30 mg/ml.

La teneur en véhicule lipidique, en particulier en huile végétale, dans la composition selon l'invention est, de préférence, de 90 à 99%, notamment de 95 à 99% (v/v) du volume total de la composition.

Les compositions selon l'invention peuvent également contenir au moins un excipient ou additif usuel dans le cas d'une composition huileuse, par exemple un co-solvant, tel que par exemple un alcool, ou un agent anti-oxydant.

Ledit co-solvant, tel que par exemple un alcool, peut être présent dans la composition, par exemple, à raison de 0 à 5%, notamment de 1 à 5% (v/v) du volume total de la composition.

En tant qu'alcool, on utilisera de préférence un alcool de formule R-OH, dans lequel R représente un groupe hydrocarboné en C₂-C₆, de préférence l'éthanol.

L'anti-oxydant peut être choisi parmi les composés usuels dans le domaine, par exemple :
- une substance hydroxylée telle que l'acide citrique et ses dérivés, notamment le citrate de sodium, de calcium ou de potassium ; l'acide ascorbique et ses dérivés, notamment, l'acide isoascorbique, l'ascorbate de sodium ou de calcium ;
- un dérivé thiol tel que, par exemple, la cystéine, l'acétylcystéine, le dithiothréitol ;
- une substance éthyléniquement insaturée, telle que l'acide sorbique, etc.

Ledit anti-oxydant peut être présent dans la composition, par exemple, à raison de 0,005 à 0,015% (p/v), de préférence 0,01%.

Un anti-oxydant préféré est choisi parmi l'acide ascorbique et ses dérivés.

Une composition préférée selon l'invention comprend 3% (p/v) de dérivé 7beta-hydroxycholestérol de formule (I), en particulier d'un des composés préférés de formule (I) mentionnés plus haut, et plus particulièrement de composé BIM2b, 0,01 % d'acide citrique anhydre (p/v) et 1% d'éthanol (v/v), dans l'huile d'argan. Ladite composition peut également être dépourvue d'éthanol.

L'invention concerne également une composition pharmaceutique comprenant une composition telle que décrite ci-dessus.

Ladite composition pharmaceutique peut également contenir un autre ingrédient actif soluble en milieu lipidique, tel que par exemple, un agent antiinflammatoire, notamment un corticoïde, ou un agent antiépileptique.

Les compositions pharmaceutiques selon l'invention peuvent se présenter, de préférence, sous une forme adaptée pour une administration par voie orale, notamment sous forme liquide, telle que, par exemple, solution buvable, suspension buvable, gouttes, etc., ou encore sous forme encapsulée, telle qu'une gélule encapsulant ladite formulation liquide, en particulier une gélule dure ou molle à base de gélatine.

L'invention concerne également un procédé de préparation d'une composition comprenant un dérivé de stérol et un véhicule lipidique telle que décrite ci-dessus, comprenant les étapes suivantes :
- la préparation d'un mélange contenant le dérivé de 7beta-hydroxycholestérol de formule (I) tel que décrit ci-dessus, un véhicule lipidique et un co-solvant,
- l'ajout éventuel d'un agent anti-oxydant, et
- le cas échéant, l'évaporation du co-solvant.

Le véhicule lipidique, l'antioxydant et le co-solvant sont tels que définis plus haut. Les aspects préférés mentionnés plus haut s'appliquent également au procédé selon l'invention.

En particulier, ledit véhicule lipidique est constitué d'une huile ou d'un mélange d'huiles, avantageusement d'une huile végétale ou d'un mélange d'huiles végétales, notamment l'huile d'argan.

Lesdites huiles, en particulier les huiles végétales, sont, de préférence, mises en oeuvre sous une forme pharmaceutiquement acceptable.

Des conditions préférées dudit procédé sont les suivantes :
- le mélange est préparé à une température de l'ordre de 22 à 35°C, de préférence 33°C ;
- la teneur en composé de formule (I) dans le mélange est de 0,1 à 3,5 % (p/v) ;
- La teneur initiale en co-solvant, de préférence l'éthanol, dans le mélange est de l'ordre de 1 à 5 %, de préférence 2,5% (v/v) ;
- la teneur en agent antioxydant, de préférence l'acide citrique et ses dérivés, dans le mélange est de 0,005 à 0,15 %, de préférence 0,01 % (p/v).

Le mélange contenant le dérivé de 7beta-hydroxycholestérol de formule (I), un véhicule lipidique et un co-solvant et, éventuellement, un anti-oxydant peut être mélangé, par exemple, à l'aide d'un évaporateur rotatif sous agitation jusqu'à dissolution du composé de formule (I).

La teneur en co-solvant, et notamment en alcool (en particulier l'éthanol) obtenue après évaporation est compatible avec la règlementation en vigueur pour les formes médicamenteuses (Règlementation ICH Q3C(R6)).

L'invention concerne également une composition comprenant un dérivé de 7beta-hydroxycholestérol de formule (I) et un véhicule lipidique, telle que définie plus haut, pour son utilisation dans le traitement d'une pathologie néoplasique, en particulier le glioblastome multiforme.

L'invention concerne également une composition comprenant un dérivé de 7beta-hydroxycholestérol de formule (I) et un véhicule lipidique, telle que définie plus haut, pour son utilisation dans le traitement d'une hémopathie maligne, en particulier une hémopathie maligne de type myéloïde.

L'invention concerne également une méthode de traitement d'une pathologie néoplasique, en particulier le glioblastome multiforme, comprenant l'administration d'une quantité efficace d'une composition comprenant un dérivé de 7beta-hydroxycholestérol de formule (I) et un véhicule lipidique, telle que définie plus haut, à un patient nécessitant ledit traitement.

L'invention concerne également une méthode de traitement d'une hémopathie maligne, en particulier une hémopathie maligne de type myéloïde, comprenant l'administration d'une quantité efficace d'une composition comprenant un dérivé de de 7beta-hydroxycholestérol de formule (I) et un véhicule lipidique, telle que définie plus haut, à un patient nécessitant ledit traitement.

L'invention concerne également une composition pharmaceutique comprenant une composition comprenant un dérivé de 7beta-hydroxycholestérol de formule (I) et un véhicule lipidique, telle que définie plus haut, pour son utilisation dans le traitement d'une pathologie néoplasique, en particulier le glioblastome multiforme, ou dans le traitement d'une hémopathie maligne, en particulier une hémopathie maligne de type myéloïde.

Les aspects généraux et préférés mentionnés plus haut, notamment pour le dérivé de 7beta-hydroxycholestérol de formule (I) et le véhicule lipidique, s'appliquent également à ces utilisations.

La dose administrée peut être, par exemple de 3 à 5 mg/kg et par jour avec une durée allant, par exemple, de 84 à 105 jours. Toutefois, la dose peut être augmentée en fonction de l'état du patient et du stade de la maladie jusqu'à 30 mg/kg/jour et la durée du traitement portée, par exemple, à 2 mois

L'invention est illustrée par les exemples 1-2 (préparation) et les exemples 3 à 8 (résultats).

Le procédé de solubilisation de BIM2b, tel que détaillé dans les exemples 1 et 2, a permis la dissolution de BIM2b dans les huiles, particulièrement celle de l'huile d'argan. Dans un aspect avantageux, à l'échelle du 1 à 500 ml d'huile, les conditions d'ajout de la poudre BIM2b à l'huile, de mélange du système BIM2b/huile et le rapport surface/volume auquel on a effectué le mélange ont conduit à une solubilisation totale de la poudre BIM2b dans l'huile.
La Figure 1 représente la chromatographie sur colonne de silice d'un extrait lipidique de cerveau de souris saine traitée par la formulation huileuse de l'exemple 2.
La Figure 2 représente la chromatographie sur colonne de silice d'un extrait lipidique de cerveau de chien souffrant de glioblastome traité par la formulation huileuse de l'exemple 2.
La Figure 3 représente le contrôle de la stabilité par HPTLC de la formulation huileuse de l'exemple 2 après les tests de résistance aux pH extrêmes.
La Figure 4 montre la régression totale de la tumeur chez un chien patient souffrant de glioblastome traité par la formulation huileuse de l'exemple 2.

Les abréviations suivantes sont utilisées :
7β-acétyl- CH : 7β-acétyl-cholestérol
7β-OHCH : 7β-hydroxycholestérol
APCI : Atmospheric Pressure Chemical Ionisation source
CCM : chromatographie sur couche mince
CH : cholestérol
CHCl3 : Chloroforme
Composé BIM2b : composé 7-acétoxy-10,13-diméthyl-17-(6-méthylheptan-2-yl)-2,3,4,7,8,9,10,11,12,13,14,15,16,17-tétradécahydro-1H-cyclopenta-[a]phenanthren-3-yl 2,2-diméthyl-1,3-dioxolane-4-carboxylate, également dénommé Cholest-5-ene-3,7-diol, 7-acetate 3-[[(4S)-2,2-dimethyl-1,3-dioxolan-4-yl]carboxylate], (3β,7β)
Composé BIM2b PO : composé BIM2b sous forme de formulation huileuse de l'exemple 2 administrable par voie orale
EtOH : éthanol
Folch : CHCl3 :MetOH (2 :1, v :v)
HA : Huile d'argan
HCl : Acide chlorhydrique
GBM : glioblastome multiple
HPTLC : chromatographie sur couche mince de silice à haute performance
KCl : Chlorure de potassium
MetOH : méthanol
NaOH : Hydroxyde de sodium
p : poids
PM : poids moléculaire
TA : température ambiante
UPLC MS : Chromatographie liquide ultra performante-spectrométrie de masse
v : volume

### A/ MOLECULES ET VEHICULE UTILISES ET ANALYSES

### 1) Principe actif

Le cholest-5-ene-3,7-diol, 7-acetate 3-[[(4S)-2,2-dimethyl-1,3-dioxolan-4-yl]carboxylate], (3β,7β) a été synthétisé au grade GLP/GMP comme décrit dans la demande WO2013/168096 (obtenu sous forme de poudre comme décrit dans l'exemple 6).

Cette molécule, dont la formule est donnée ci-dessous, est nommée BIM2b dans toute la partie expérimentale.

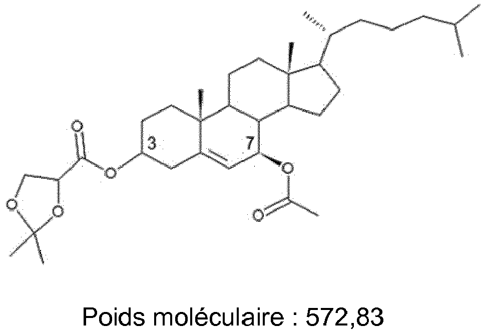

### 2) Produits de dégradation et métabolites majeurs du principe actif

a) Le 7β-acétyl-cholestérol a été synthétisé comme décrit dans la demande WO2013/168096 (composé 1.4).
   Cette molécule, dont la formule est donnée ci-dessous, est nommée 7β-acétyl-CH dans toute la partie expérimentale. 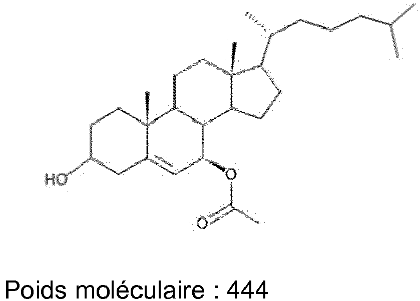
b) Le 7β-hydroxycholestérol a été fourni par SIGMA (référence 700035P).
   Cette molécule, dont la formule est donnée ci-dessous, est nommée 7β-OHCH dans toute la partie expérimentale. 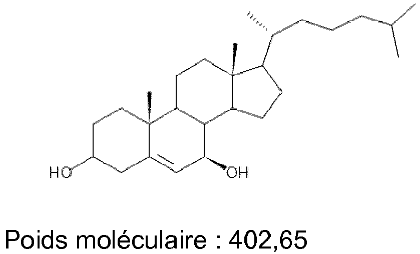
c) Le cholestérol a été fourni par SIGMA (référence C8503).
   Cette molécule, dont la formule est donnée ci-dessous, est nommée CH dans toute la partie expérimentale. 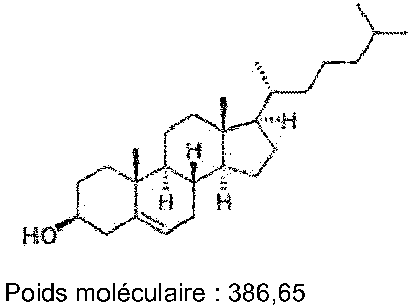
d) Le véhicule choisi est l'huile d'argan (Olsana, France), nommée HA dans toute la partie expérimentale.

### B) MATERIEL ET METHODES

### I) MATERIEL

### I.1) Chromatographie

### I.1.1) Chromatographie d'adsorption sur couche mince de silice à haute performance (HPTLC, High Performance Thin Laver Chromatography) et sur colonne de silice classique

- Cuves multiplaques pour chromatographie 20 x 20 cm (référence 552-0359)
- Colonnes de chromatographie avec verre fritté (longueur utile : 300mm ; diamètre int.= 11mm ; robinet PTFE clé 1,5mm) (Analytic Lab, référence LE-AT1811)
- Gel de silice 60 en poudre (0,063-0,200mm) (référence 1.07734.1000)
- Plaque de HPTLC : silice gel 60 F254 avec zone de concentration (2,5 cm) sur feuille d'aluminium (VWR, référence 1055830001).
- Nébuliseur et erlenmeyer 100ml (Witeg, référence 2147100, NS 14,5/23)
- Plaque chauffante (Stuart SB162)
- Tube en verre borosilicaté
- Scanner (Epson Perfection V370 Photo)
- Microcapillaires Drummond 10µl (Sigma, référence P1924)

### I.1.2) Chromatographie sur cartouche d'adsorption ou de phase inverse

- SEP PAK Classic silica (Waters, référence WAT051900)
- SEP PAK Plus C18 (Waters, référence WAT023635)

### I.1.3) Chromatographie liquide ultra performante - Spectrométrie de masse (UPLC - MS)

La mise au point de la quantification des molécules étudiées (BIM2b et ses métabolites) a été effectuée à l'aide d'un système Nexera X2 UPLC (Shimadzu) équipé d'une colonne Kinetex EVO C18 (taille des particules: 1,9µm ; dimensions de la colone : 100 x 2,1 mm, Phenomenex). La détection APCI se fait avec un quadrupole (LCMS 8050, Shimadzu).

### I.2) Formulation de BIM2b pour une administration par voie orale (BIM2b PO)

- Balance de précision Sartorius Quintix (Sartorius, référence QUINTIX 124-1S)
- Tubes en verre 5ml borosilicaté 12 x75 mm (Kimble, référence 73500-1275)
- Tubes en verre 25ml borosilicaté pyrex 18 x 180 mm (Dutscher, référence 090482)
- Becher de 5 ml
- Ballon réactionnel de 1l à bouchon rôdé (femelle, 29/32NS) (VWR, référence 201-1359)
- Evaporateur rotatif R-215 Buchi
- Microscope optique inversé à contraste de phase Eclipse TS-100F trinoculaire Nikon (Dutscher, référence : 094501) couplé avec une caméra digitale C-mount (NIKON, DCMC510)

### I.3) Quantification des molécules étudiées (BIM2b et ses métabolites): homogénéisation de cerveaux de souris sauvages et de tumeurs issues après nécropsie de cerveaux de chiens souffrant de GBM

- Homogénéiseur (Ika eurostar 20 digital)
- Broyeur de tissu type Potter-Elvehjem (capacité: 4ml ; longueur: 120 mm ; diamètre extérieur: 11 mm) (VWR, référence 432-0200)
- Ampoule à décanter sphérique 250 ml avec robinet en PTFE (Dutscher, référence 479036)
- Ampoule à décanter 50 ml avec robinet PTFE (Dutscher, référence 084118)

### I.4) Solvants organiques et autres consommables

- Silice pour colonne de chromatographie silica gel 60 (0,063-0,200mm) (Merck, référence 1.07734.1000)
- CHCl₃ grade HPLC (Sigma, référence 528730)
- EtOH absolu (Sigma, référence SI-32221)
- MetOH chromasolv pour HPLC ≥ 99,9% (SIGMA, référence 34860)
- N-Hexane pour HPLC (Fisher scientific, référence 1070361)
- Diéthyloxyde stabilisé pour HPLC (Analytic Lab, référence FI-10254914)
- Acétone (Sigma-Aldrich, référence 322201)
- Acide orthophosphorique 99% (Analytic lab, référence U1B203102H)
- Acétate de cuivre 98% (Sigma, référence 326755/
- Hydroxytoluène butylé (BHT ; Sigma, W218405, référence W218405)
- Acide chlorhydrique min 37% (HCl, Thermofisher, référence M0643H)
- Soude (NaOH/Thermofisher, référence 04815)
- Carbonate d'ammonium (Sigma, référence 207861)
- Chlorure de potassium (Fisher Scientific, référence W2752)
- Formol ou formaldehyde en solution (36,5-38% dans l'eau) (Sigma, référence F8775)
- Ammonium acétate (Merck, référence C138315)

### II) Méthodes

### II.1) Chromatographie HPTLC

Cette approche analytique a été utilisée pour quantifier BIM2b, ses produits de dégradation et ses métabolites majeurs dans le médicament (BIM2b PO) et des cerveaux de souris sauvages ou des tumeurs GBM issues de chiens qui ont souffert de cette pathologie. Le seuil de détection de BIM2b et de ses produits de dégradation (7β-acétyl-CH et 7β-OHCH) déterminé expérimentalement est de 0,5 µg.

Cette méthode a été validée par la méthode UPLC - MS.

### - Traitement des plaques HPTLC

Les cuves multiplaques de chromatographie sont saturées avec le système d'éluant le mélange CHCl₃ : MetOH (1 :1 ; v:v) pendant 1 h. Les plaques HPTLC sont placées dans les cuves ; l'élution est arrêtée lorsque le front d'éluant arrive à 1,5-2 cm du bord haut des plaques. Par la suite, elles sont séchées à température ambiante pendant 1 h. Ensuite, ces plaques sont activées à 110°C pendant 15 min. Une fois refroidies, elles sont enveloppées dans des feuilles d'aluminium et stockées à l'abri de la lumière et de l'humidité.

### - Dépôts des échantillons sur les plaques HPTLC et conditions chromatographiques

Tous les échantillons sont solubilisés dans de l'acétone et déposés avec des capillaires drummond (10 µl) sur la zone de de concentration des plaques HPTLC. Lorsque les gammes étalons de BIM2b, du 7β-acétyl-CH et du 7β-OHCH sont utilisés elles sont préparées à partir de solutions mères éthanoliques de 0,1 mg/ml pour chaque molécule-étalon

La séparation chromatographique s'effectue en deux étapes : une étape de pré-élution (3 fois) avec comme système d'éluant le mélange CHCl3 :MetOH (2 :1, v/v) pour la zone de concentration, suivie d'une étape de séparation (3 fois) pour la zone de séparation conduite avec comme système d'éluant le mélange Hexane :Ether (3:7, v/v) afin d'individualiser et de quantifier les stérols.

Avant la chromatographie, les cuves sont saturées avec les systèmes d'éluant respectifs à la concentration et à la séparation.

Entre chaque élution les plaques sont séchées à l'aide d'un flux froid émanant d'un sèche-cheveux.

### - Révélation et quantification des oxystérols

Les oxystérols sont révélés selon la méthode de Macala (1). Le réactif de Macala consiste en une solution aqueuse à 8% (p/v) d'acide orthophosphorique et à 3% (p/v) d'acétate de cuivre. Suite à la chromatographie, les plaques HPTLC sont séchées, pulvérisées par le réactif de Macala et chauffées sur une plaque chauffante à 140°C. Les oxystérols se colorent en bleu de Prusse et le cholestérol en mauve. Les spots colorés sont scannés et ensuite quantifiés par Image J (version 1.50g).

La quantification des oxystérols et du cholestérol par la méthode HPTLC a été validé par la méthode UPLC - MS.

### II.2) UPLC - MS

### Conditions expérimentales d'élution et de détection :

### - Quantification de BIM2b et du 7β-OHCH

L'élution isocratique se fait avec une phase mobile composée de MetOH contenant 5mM d'acétate d'ammonium.

### - Quantification de 7β-acetyl-CH :

L'élution se fait avec un gradient binaire linéaire. L'éluant A étant 0.1% (v :v) d'acide formique et l'éluant B du MetOH contenant 0.1% d'acide formique. L'élution commence avec un mélange (v :v) de 20% A et 80%B pour finir avec 100% de l'éluant B ; la durée du gradient est de 3 min.

Le volume des injections est de 10µl et la détection se fait par APCI en mode positif.

### II.3) Extraction et purification de BIM2b et de ses produits de dégradation

Excepté la quantification de BIM2b, du 7β-acétyl-CH et du 7β-OHCH lors des tests de stabilité de BIM2b dans BIM2b formulé (BIM2b PO) où les échantillons de BIM2b PO sont appliqués directement sur les plaques HPTLC, une extraction et une purification des oxystérols est nécessaire pour quantifier ces molécules dans BIM2b PO exposé à des conditions de pH extrêmes, dans des cerveaux de souris sauvages gavées par voie orale par BIM2 PO ou dans des tumeurs GBM issus de nécropsie d'un chien souffrant de GBM survenu spontanément (patient chien GBM), traités par voie orale par BIM2b et ayant survécu pendant 8 mois (la survie moyenne d'un chien atteint de GBM est de 2 - 3 mois malgré des traitements de chimiothérapie).

L'extraction des oxystérols s'effectue selon la méthode préconisée par Folch et al. (2). L'extraction se déroule dans une ampoule à décanter. Brièvement, l'extraction se fait avec un mélange CHCl3 :MetOH (2 :1, v :v ; Folch) ; pour cette méthode, le rapport volume de Folch/volume de l'échantillon aqueux est de 19. Rincer et casser la phase avec 0.2 volume de la phase organique de solution aqueuse contenant du KCl à 0,74 %.

Pour le cas de l'extraction des oxystérols de cerveaux de souris saines traitées par voie orale par BIM2b PO ou d'une tumeur GBM de chien lui aussi traité par voie orale par BIM2b PO, la phase organique rincée par 0.74% KCl est encore une fois rincée par 0.4 volume du mélange CHCl3 : MetOH:H20 (3:48:47 ; v :v :v ; phase supérieure de Folch). Le résidu séché est ensuite solubilisé dans un solvant approprié au procédé expérimental qui suit l'étape d'extraction.

### II.4) Échantillons nécessitant une extraction avant la séparation et la quantification des oxystérols

### - Test de résistance de BIM2b PO à un pH acide extrême suivi d'une incubation à un pH basique.

Pour ces tests, BIM2b PO est d'abord incubé d'abord dans un bain acide à pH 1,5 et ensuite dans un bain basique à pH 8,5. Ces 2 valeurs correspondent respectivement aux pH physiologiques de la digestion gastrique et à celui de la digestion au niveau de l'ampoule de Vater chez l'homme.

Le bain aqueux acide est préparé à partir de solutions stock HCl 1M et 0.1M. Le bain basique est une solution aqueuse de carbonate d'ammonium à 25 mM.

Un premier becher contenant 4 ml de bain acide (pH 1,5) est chauffé à 37°C avant de démarrer l'expérience ; 2 ml de BIM2b PO sont ensuite doucement déposés à la surface du bain. Le mileu réactionnel est agité par un barreau magnétique pendant 10 h. Après arrêt de l'agitation, on laisse reposer le milieu réactionnel pendant 2 h afin que la phase supérieure huileuse soit bien reformée. Durant les 2 h de repos du milieu réactionnel, le bain basique (pH 8,5) est préparé dans un deuxième becher et chauffé à 37°C. On prélève 1 ml de la phase lipidique du bain acide que l'on dépose doucement à la surface aqueuse du bain basique. On démarre l'agitation pour 10 h d'incubation. Dès l'arrêt de l'agitation, la totalité du mélange (BIM2b PO et le bain basique) est soumise à extraction de Folch sans relaver la phase organique avec la phase supérieure de Folch.

### - Extraction et quantification des oxystérols et de leurs métabolites à partir de cerveaux de souris saines traitées par BIM2b PO (voie orale)

On laisse les cerveaux de souris séchés et conservés invididuellement dans des cryotubes à 180°C dans l'azote liquide revenir à température ambiante. Chaque cerveau est pesé et introduit dans le potter ; 4ml de 0.74% de KCl froid sont ajoutés et la suite du procédé se déroule sur lit de glace. Chaque cerveau est broyé 6 fois à 1000 tours/min, chaque cycle comprenant 5 minutes de broyage et 5 minutes de repos. Les oxystérols sont extraits selon la procédure de Folch décrit ci-dessus (II.3.). Le résidu lipidique est ensuite soumis à la chromatographie d'adsorption sur colonne de silice.

La silice (8 g) est préparée dans 40 ml du mélange hexane :ether (diethyl-oxide (3 :7, v/v) et ensuite coulée dans les colonnes appropriées. La hauteur du lit de silice est de 18 cm et son volume est de 15 ml.

Une quantité appropriée d'extrait de cerveau de souris est dissoute dans 1 ml du premier système d'éluant et déposé sur le lit de silice. L'élution se fait en 4 étapes afin de de concentrer les fractions contenant les oxystérols et d'éliminer le plus possible les lipides contaminant du cerveau de souris, en particulier le cholestérol.

Les élutions se déroulent comme suit :
1^{ère} élution : hexane :ether (3 :7, v :v)
2^{ème} élution : Acétone :EtOH (95 :5, v :v)
3^{ème} élution : Acétone :EtOH (93 :7, /v)
4^{ème} élution : EtOH

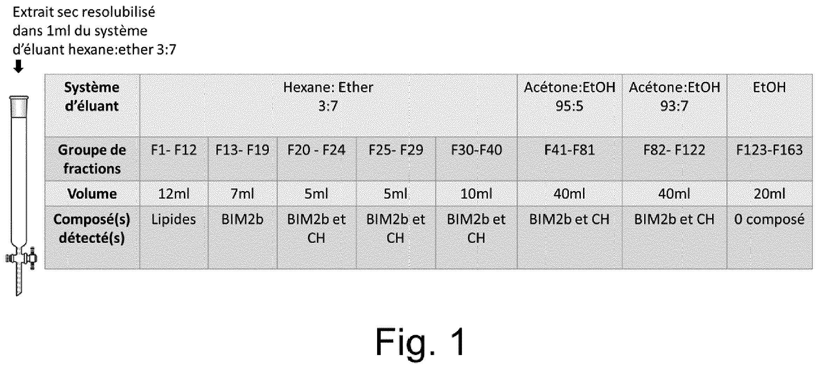

La marche de l'élution est décrite sur la Figure 1.

Les groupes de fractions sont évaporés à sec, dissous dans de l'acétone et les volumes appropriés sont soumis à la chromatographie HPTLC.

### - Extraction et quantification de BIM2b et de ses métabolites à partir d'une tumeur GBM de chien traité par BIM2b PO (voie orale)

La tumeur GBM a été obtenue par nécropsie d'un chien souffrant de glioblastome qui a été traité par voie orale par BIM2b PO et qui a vécu dans de bonnes conditions de vie durant 8 mois. La nécropsie a été effectuée 21 jours après l'arrêt du traitement par BIM2b PO. La tumeur a été fixée dans du formol principalement pour l'analyse d'histo-pathologie et un fragment restant a été analysé pour la quantification de BIM2b.

Sur des fragments de cerveaux de chiens ne souffrant pas de GBM et sur une partie du fragment tumoral, tous les contrôles ont été faits pour démontrer que la fixation par le formol n'altérait ni la qualité des oxystérols, ni le rendement de leur extraction par la technique de Folch.

Les fragments de cerveaux de chiens, conservés individuellement dans des cryotubes à +4°C dans un bain de formol, sont séchés sur du papier sopalin avant d'être pesé et introduit dans le potter. Comme pour l'extraction des cerveaux de souris, chaque fragment a été extrait après ajout de 4 ml de 0,74% de KCl froid. La procédure d'homogénéisation et d'extraction sont les mêmes que celles décrites pour les cerveaux de souris dans la section Méthodes.

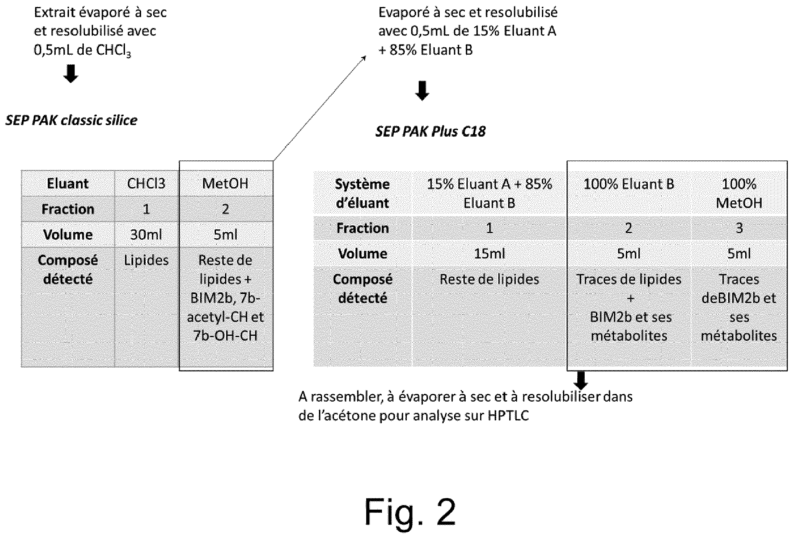

De l'extrait sec lipidique du fragment tumoral GBM de chien est ainsi obtenu et a été chromatographié séquentiellement sur cartouche de SEP PAK classic silice ; puis sur SEP PAK Plus C18 (avec les éluant A = 5 mM d'acétate d'ammonium/eau ultrapure et B = 5 mM d'acétate d'ammonium/ MetOH). Le processus de chromatographie sur colonnes SEP PAK est représenté sur la Figure 2.

### C. Exemples de préparation et résultats

### Exemple 1 :Essais de véhicules lipidiques

Les huiles testées sont l'huile d'argan, l'huile d'avocat, l'huile d'olive, l'huile d'arachide, l'huile de pépin et l'huile de raisin. Les tests ont été faits à l'échelle du tube à essai.

Différentes quantités de BIM2b, allant de 1 à 35 mg, ont été ajoutées à 1 ml de l'huile de choix, en plusieurs fois, pour atteindre la quantité déterminée. Entre chaque ajout de BIM2b, le tube à essai est agité doucement.

Les tubes sont placés pendant 24 h dans un incubateur à 37°C. Durant les 5 premières heures d'incubation, les tubes ont été sortis chaque heure de l'incubateur afin de les agiter doucement et replacés dans l'incubateur durant toute la nuit. Le lendemain, l'aspect du mélange de l'huile avec BIM2b a été vérifié visuellement pour estimer le degré de dissolution de la poudre dans l'huile. Lorsque BIM2b semble totalement dissous dans l'huile à l'oeil nu, un examen microscopique est fait pour vérifier la présence ou non de BIM2b sous forme de poudre ou de cristaux.

Les résultats montrent que BIM2b se dissout totalement dans l'huile d'avocat, l'huile d'olive, l'huile d'arachide, l'huile de pépin et l'huile de raisin à une concentration de 3 mg de BIM2b/ml d'huile.

Dans l'huile d'argan (HA), BIM2b est dissout jusqu'à une concentration de 30 mg (52µmoles) de BIM2b/ml de HA.

### Exemple 2 : Préparation de BIM2b PO

On a utilisé comme récipient de fabrication un ballon de 1l dans lequel on introduit 285 ml d'huile d'argan (Olsana, France). On incline la position du ballon et on le fait tourner manuellement autour de lui-même afin de répartir l'huile de manière homogène sur la paroi du ballon selon un rapport surface/volume: 1,6 cm ⁻¹ (Résultat du calcul : 460 cm²/285 cm³). En maintenant cette position, on incorpore 9 g de poudre BIM2b sur toute la paroi de manière à recouvrir toute la surface du ballon. Par la suite, on ajoute 3 mL d'éthanol contenant 30 mg d'acide citrique (à partir d'une solution-stock à 10 mg d'acide citrique/ml d'éthanol); puis 12 ml d'éthanol (soit 5% d'éthanol). On place le ballon sur un évaporateur rotatif oblique Buchi® R-215 dans un bain-marie à 30°C à la vitesse de 15 rpm pendant 2 h. Pour évaporer l'éthanol, on laisse ensuite évaporer à bouchon ouvert dans un bain-marie à 30°C pendant une nuit.

On obtient ainsi 300 ml de composition contenant le BIM2b dans l'huile d'argan à une concentration de 30 mg/ ml.

La formulation BIM2b PO ainsi obtenue, qui sera utilisée dans les exemples qui suivent, contient 3% (p/v) de BIM2b, 0,01% (p/v) d'acide citrique anhydre et 1% d'EtOH (v/v) (après évaporation) dans l'huile d'argan.

La quantification de BIM2B PO et de ses produits de dégradation juste après la fabrication de ce lot montre une concentration de 30 mg de BIM2b/ml de BIM2b PO sans aucun produit de dégradation.

Une étude de stabilité des lots BIM2b PO fabriqués selon ce procédé montre, dans les condition expérimentales, la présence de 1% de produits de dégradation (p/v) au bout de 6 mois de conservation et inférieure ou égale à 2% (p/v) au bout de 12 mois de conservation du même lot.

### Exemple 3 : Test de résistance de BIM2b PO à un pH acide suivi d'une incubation à un pH basique

Le déroulement du test est décrit plus haut dans la section II) Méthodes.

Après incubation de BIM2b PO dans les bains successifs acide (pH 1,5) et basique (pH 8,5) ; l'extrait lipidique sec a été dissous dans l'acétone. Pour des raisons d'encombrement (4 pistes à 15,30, 40 et 60 µg théoriques), on n'a pas déposé de gamme-étalon.

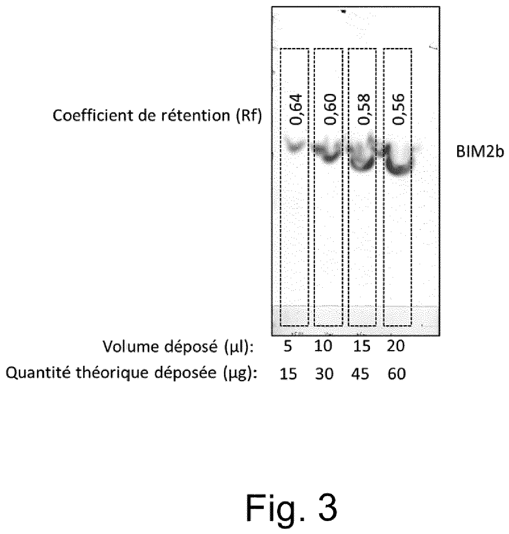

Les résultats analytiques sur HPTLC rapportés sur la Figure 3 montrent la détection d'une bande unique, celle de BIM2b, quelle que soit la quantité déposée, avec un coefficient de rétention (Rf) de 0,5 à 0,64. Les Rf des produits de dégradation 7β-acétyl-CH et du 7β-OHCH (non visibles) déterminés expérimentalement sont respectivement, de 0,37 et 0,24 Ces résultats montrent que BIM2b PO résiste à des conditions de pH extrêmes sans être dégradé.

### Exemple 4 :Étude de toxicité de BIM2b PO chez la souris saine

On a administré par voie orale à des souris sauvages (par gavage) la composition de l'exemple 2 à raison de 1,5 mg (50 µl de BIM2 PO) par animal, 2 fois par jour, 5 jours sur 7 avec une durée maximale de traitement de 21 jours. Parallèlement, un groupe témoin de souris sauvages n'a reçu aucun traitement.

Les échantillons d'organes internes (estomac, tube digestif, foie, rein, poumon, coeur) ont été immergés dans du formaldéhyde à 4% pendant 72 h et stockés à 4°C, puis implantés dans la paraffine. Les échantillons ont été soumis à une analyse macroscopique avant et après l'amincissement.

Les analyses histologiques des organes internes fixés par le formaldéhyde et les quantifications toxicologiques ont été réalisées sur des coupes colorées à l'hématoxyline éosine safran selon un système d'évaluation sur 4 niveaux conformes à la nomenclature (3).

Les signes éventuels d'altération cellulaire, dégénération, nécrose, inflammation, nécrose, fibrose etc. ont été recherchés sur toutes les coupes.

Les conclusions sont les suivantes (pour chaque organe) :
- Estomac : aucune lésion chez aucune souris traitée
- Intestin grêle : inflammation minimale de nature lymphoplasmacystique interprétée comme faisant partie du bruit de fond. Pas de lésion significative.
- Gros intestin : aucune lésion chez aucune des souris.
- Foie : une tendance à la baisse du stockage cytoplasmique et une hypertrophie hépatocellulaire ont pu être observées chez les souris traitées par comparaison aux souris témoins. Ce changement est resté très diffus et ne peut être attribué avec certitude au traitement.
- Rein : pas de lésion significative détectée chez aucune des souris.
- Poumon : histiocytose minimale et légère inflammation alvéolaire chez l'une des souris traitées, peut-être causées par l'alimentation orale forcée.
- Coeur : aucune lésion détectée chez aucune des souris.

En conclusion, les résultats montrent que la molécule BIM2b n'entraîne aucune toxicité. L'administration de BIM2b à la posologie indiquée, 2 fois par jour, 5 fois par semaine, avec absence de traitement le week-end n'a pas entraîné d'effets indésirables cliniques. De même, les paramètres biologiques étudiés n'ont pas mis en évidence de toxicité hépatique ou rénale. Le diabète et l'hypercholestérolémie auxquels on pouvait s'attendre avec l'administration d'un stéroïde n'ont pas été observés.

### Exemple 5 : Étude de tolérance de BIM2b PO chez le chien sain

On a administré par voie orale à 3 chiens Beagle d'un poids moyen de 11 kg la composition de l'exemple 2 à raison de 15 mg (0,5 ml), 2 fois par jour (le matin après le repas du jour et le soir à jeun), 5 jours sur 7 avec une durée de traitement de 21 jours.

Le calcul de la dose administrée était basé sur la consigne d'administration de 3 mg/kg et par jour.

Un examen clinique complet des animaux a été réalisé quotidiennement en semaine de J0 jusqu'à J21 : pesée, prise de température rectale, état général, appétit. Le week-end (période de repos sans administration), les animaux n'ont fait l'objet que d'une observation simple afin de vérifier que leur état général et leur comportement étaient normaux.

Des prélèvements de sang pour numération formule sanguine (NFS) et dosages biochimiques ont été réalisés au début et en fin de traitement (à J0 et J21). Tous les chiens ont pris la totalité du traitement. Aucun effet secondaire n'a été observé lors de l'examen des chiens.

Les résultats de l'étude sont les suivants :
L'utilisation de stéroïdes pouvant entrainer une augmentation du poids, les chiens ont été pesés tous les jours du traitement. Contrairement à ce qui était attendu, on a observé une perte de poids sur l'ensemble des chiens d'environ 4,5%.

Aucune augmentation du taux de cholestérol et de triglycérides n'a été mise en évidence. Au contraire, il a été mesuré une diminution de la quantité de ces molécules : -2,3% en moyenne pour le cholestérol, -30% en moyenne pour les triglycérides.

L'utilisation de stéroïdes peut également être source d'apparition de diabète. Aucune hausse de la glycémie n'a été observée à J21. Une baisse du glucose de 12,9% en moyenne a, au contraire, été observée.

Au vu du bilan rénal effectué (urée et créatinine), aucune toxicité rénale n'a été observée chez les 3 chiens.

Le bilan hépatique (albumine, protéines, aspartate aminotransférase (AST) et alanine aminotransférase (ALT) (ratio AST/ALT), phosphatase alcaline, bilirubine totale) des 3 chiens était normal après traitement. Aucune variation significative dans les paramètres hématologiques et biochimiques n'a été observée par ailleurs.

En conclusion, le composé BIM2b administré sous forme de composition BIM2b PO n'entraine aucune toxicité. L'administration de cette composition à la posologie de 30 mg/ml de composé BIM2b, 2 fois par jour, 5 jours par semaine, pendant 21 jours n'a pas entrainé d'effets indésirables cliniques. De même, les paramètres étudiés n'ont pas mis en évidence de toxicités hépatiques ou rénales.

### Exemple 6 : Etude de l'efficacité anti - GBM de BIM2b PO administrée par voie orale à des patients chiens atteints de gliomes spontanés (dénommés « patients chiens GBM »)

Cette étude avait pour but d'évaluer l'efficacité du composé BIM2b administré dans une composition selon l'invention dans le traitement de gliomes canins.

9 chiens atteints de gliomes spontanés diagnostiqués en imagerie ont été inclus dans l'étude. Les chiens ont été traités par voie orale avec une dose quotidienne de 3 mg/kg par tranche de 5 kg de composé BIM2b (1ml de composition de l'exemple 2).

L'étude comprend 5 cycles de traitement de 21 jours (105 jours) suivis par 2 cycles de 3 mois d'observation. L'état et le volume tumoral sont évalués par imagerie IRM.

Au jour J0 (1^{ère} administration), le patient chien GBM est examiné cliniquement et une prise de sang est effectuée pour analyser les paramètres biochimiques. A J15, on effectue un électrocardiogramme et une prise de sang. A partir de J21, la dose est revue à la hausse (+ 25% de la dose originale) ou à la baisse (- 25% de la dose originale) en fonction de la réponse tumorale et/ou des toxicités.

Des électrocardiogrammes, prises de sang et imageries IRM sont réalisés à J42, J63, J84 et J105, puis 3 mois et 6 mois après l'arrêt du traitement.

La réponse tumorale est évaluée par imagerie IRM au moyen des critères RECIST (Response Evaluation Criteria In Solid Tumors). Ces critères définissent la réponse tumorale comme suit :
- la progression est une augmentation de taille du plus grand axe de la tumeur de plus de 20% par rapport à l'IRM précédent ;
- la régression est définie par une diminution de la taille du plus grand axe de la tumeur de plus de 30% par rapport à l'IRM précédent ;
- la stabilité est définie par une variation de la taille de la tumeur comprise entre une diminution de moins de 30% et une augmentation de moins de 20%.

### Résultats

Cette étude s'est déroulée sur 22 mois. 3 des 9 chiens inclus n'ont pas été analysés, car n'ayant pas atteint J42 (décès dûs, notamment,à une inclusion à un stade trop avancé de la maladie). Parmi les 6 chiens restants, 2 chiens (**N** et G) sont décédés à J165 (5,5 mois) et à J126 (4,2 mois) respectivement. C'est la tumeur du patient chien GBM **N** qui a été utilisée pour quantifier BIM2b et ses métabolites (exemple 8).

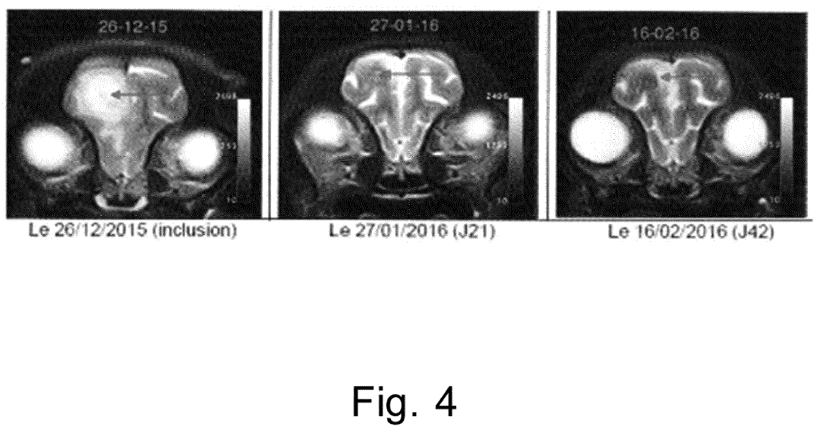

Pour les 4 patients chiens GBM restants, il a été constaté que le traitement par voie orale est bien toléré. Une amélioration des signes cliniques est notée pour les 4 chiens : 2 sont restés stables et une très nette régression a été observée dans les deux autres cas avec quasiment une disparition de la lésion pour l'un d'entre eux (**D**). La Figure 4 montre la disparition de la tumeur pour le chien **D** à J42.

### Exemple 7 : Analyse de BIM2b dans les cerveaux de souris traitées per os par BIM2b PO

Le traitement des souris saines per os par BIM2b PO et la provenance des cerveaux sont décrits dans l'exemple 4.

Le composé BIM2b et ses métabolites dans les cerveaux sont séparés, détectés et quantifiés par HPTLC comme décrit dans la partie méthodes.

Les résultats sont donnés dans le tableau 1 ci-dessous :

**Tableau 1**

| Jour | Poids du cerveau (mg) | Composé BIM2b (µg/g de cerveau) |
|---|---|---|
| J2 | 415 | 12,5 |
| J4 | 490 | 7,1 |
| J18 | 471 | 11,7 |
| J22 | 466 | 1,0 |

Les résultats montrent que le composé BIM2b est retrouvé dans les cerveaux de souris traitées par voie orale par BIM2b PO dans une composition selon l'invention. Ceci démontre que le composé BIM2b traverse la barrière hémato-encéphalique (BHE) des souris saines traitées. On observe à J22 une corrélation évidente entre la chute remarquable (91,5%) de la teneur en BIM2b dans le cerveau de souris et l'arrêt de traitement à J20. Les métabolites ne sont retrouvés que sous forme de traces.

Dans le cas d'une pathologie néoplasique, on ne parle pas de BHE mais de BHT (barrière hémato-tumorale). La BHT est fragilisée par la présence de la tumeur et par sa physiologie. Les recherches montrent que les molécules traversent plus facilement la BHT que la BHE.

Le passage de la BHE peut donc être considéré comme prédictif du passage de la BHT.

Le pourcentage de BIM2b retrouvé dans les cerveaux, par rapport à la quantité totale que les souris ont ingérée, est de 0,013% à J18.

### Exemple 8 : Analyse de BIM2b dans une tumeur de patient chien GBM traitée par voie orale par BIM2b PO.

La tumeur GBM a pour origine le chien N de l'exemple 6.

Le composé BIM2b et ses métabolites dans la tumeur sont séparés, détectés et quantifiés par HPTLC comme décrit dans la partie II) Méthodes.

Les résultats montrent la présence de 3,7 µg de BIM2b dans les 212,7 mg de fragment tumoral analysé. Le pourcentage pondéral de BIM2b retrouvé dans toute la tumeur par rapport à la quantité totale de BIM2b que le chien a ingérée est de 0,39% ; soit 30 fois plus que pourcentage pondéral retrouvé dans les cerveaux de souris saines (exemple 7).

Le fait que la nécropsie de la tumeur ait été effectuée 21 jours après l'arrêt du traitement et que du BIM2b soit quand même retrouvé dans la tumeur montre que le composé BIM2b contenu dans BIM2b PO traverse la BHT et se retrouve préférentiellement dans la tumeur GBM.

### Références

1. Macala, LJ, Yu RK and Ando, S. J. Lip. Res. (1983), 24: 1243 -1250.
2. Folch, J, Lees, M and Sloane-Stanley, GH. J. Biol. Chem. (1957), 226 : 497-509.
3. Mann PC, Vahle J., Keenan, CM, Baker JF, Bradley, AE, Goodman, DG, Harada,T, Herbert, R, Kaufmann, W, Kellner, R, Nolte, T, Rittinghausen, S, and Tanaka, T. Toxicologic Pathology (2012), 40 (4 Suppl).

## Revendications

1. Composition comprenant au moins un dérivé de 7beta-hydroxycholestérol et un véhicule lipidique, à l'exclusion d'un véhicule lipidique consistant en des vésicules lipidiques formées d'une ou plusieurs couche(s) lipidique(s), dans laquelle le dérivé de 7beta-hydroxycholestérol répond à la formule (I) 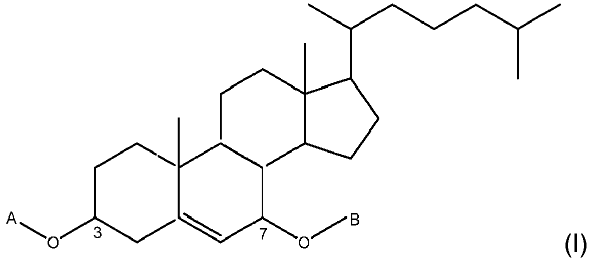 dans laquelle :
- A représente
un groupement -C(O)R₁ dans lequel R₁ est un hétérocycle saturé comprenant 5 à 14 membres et incluant 1 ou 2 hétéroatomes, non substitué ou substitué par au moins un alkyle en C₁-C₆ linéaire ou ramifié ou un groupement choisi parmi OR, NRR', NHR et SR, où est R et R', indépendamment, représentent l'hydrogène, un alkyle en C₁-C₁₂, de préférence en C₁-C₆, linéaire ou ramifié, ou un aryle non substitué ;
ou un groupement -(R₂)ₙ- dans lequel R₂ est un reste d'acide aminé lié par son extrémité C-terminale et n= 1 à 3, chacun des R₂ étant identique ou différent, dans lequel l'extrémité N-terminale dudit acide aminé peut être substituée par un groupe -C(O)R₃ dans lequel R₃ est un groupe arylalkyl mono-ou pluri-cyclique en C₆-C₁₄ ; un groupe hétéroarylalkyl mono-ou pluri-cyclique en C₅-C₁₄ pouvant comporter un ou plusieurs hérétoatomes, identiques ou différents; un groupe arylalkyloxy mono-ou pluri-cyclique en C₆-C₁₄ ou un groupe hétéroarylalkyloxy mono-ou pluri-cyclique en C₅-C₁₄ pouvant comporter un ou plusieurs hérétoatomes, identiques ou différents,
- B représente un groupement -C(O)R₄, dans lequel R₄ est un alkyle en C₁-C₁₂, de préférence en C₁-C₆, linéaire ou ramifié, non substitué ou substitué par un groupement choisi parmi OR, NRR', NHR et SR, tels que définis ci-dessus; un groupe aryle, non substitué ou substitué par un groupement choisi parmi OR, NRR', NHR et SR, tels que définis ci-dessus; ou encore R₄ représente OR₅, dans lequel R₅ est un alkyle en C₁-C₁₂, de préférence en C₁-C₆, linéaire ou ramifié.

2. Composition selon la revendication 1, **caractérisée en ce qu'**elle se présente sous une forme adaptée pour une administration par voie orale.

3. Composition selon la revendication 1, **caractérisée** en ce le composé de formule (I) est en solution dans ledit véhicule lipidique.

4. Composition selon l'une des revendications 1 à 3, **caractérisée en ce que**, dans la formule (I), A représente un groupement -C(O)R₁ dans lequel R₁ est un hétérocycle saturé comprenant 5 membres et incluant 2 atomes d'oxygène, substitué par au moins un alkyle en C₁-C₆ linéaire ou ramifié.

5. Composition selon l'une quelconque des revendications 1 à 4, **caractérisée en ce que**, dans la formule (I), B représente un groupement -C(O)R₄ dans lequel R₄ est un alkyle en C₁-C₆, linéaire ou ramifié ou R₄ représente OR₅, dans lequel R₅ est un alkyle en C₁-C₁₂, de préférence en C₁-C₆, linéaire ou ramifié.

6. Composition selon l'une quelconque des revendications 1 à 4, **caractérisée en ce que**, dans la formule (I), B représente un groupement acyl dans lequel le groupe alkyl est en C₁-C₆ ou un groupement alcoxycarbonyl dans lequel le groupe alkyl est en C₁-C₆.

7. Composition selon l'une quelconque revendications 1 à 3, 5 ou 6, **caractérisée en ce que**, dans la formule (I), A représente :
- un groupement -(R₂)ₙ- dans lequel R₂ est un reste d'acide aminé et n = 2 ; ou
- un groupement -(R₂)ₙ- dans lequel R₂ est un reste d'acide aminé, n= 2 et l'extrémité N-terminale dudit acide aminé est substituée par un groupe arylalcoxycarbonyl, en particulier benzyloxycarbonyl ; ou
- un radical alanyl lié à un radical glycinyl , éventuellement substitué sur son extrémité N-terminale par un groupe arylalcoxycarbonyl, en particulier benzyloxycarbonyl; ou
- un radical méthionyl lié à un radical glycinyl, éventuellement substitué sur son extrémité N-terminale par un groupe arylalcoxycarbonyl, en particulier benzyloxycarbonyl, et
- B est tel que défini dans l'une quelconque des revendications 1 à 3, 5 ou 6.

8. Composition selon l'une quelconque des revendications 1 à 7, dans laquelle le dérivé de 7beta-hydroxycholestérol est choisi parmi les composés de de formule (I) dans laquelle :
- A est un groupement 3-benzyloxycarbonyl-glycinyl-alanyl et B est un groupe tert-butoxycarbonyl, ou
- A est un groupement 3-benzyloxycarbonyl-glycinyl-alanyl et B est un groupe acétyl, ou
- A est un groupement -C(O)R₁ dans lequel R₁ est un groupe 2,2-diméthyl-1,3-dioxolane et B est un groupe tert-butoxycarbonyl.

9. Composition selon l'une quelconque des revendications 1 à 7, dans laquelle le dérivé de 7beta-hydroxycholestérol est un composé de formule (I) dans laquelle A est un groupement -C(O)R₁ dans lequel R₁ est un groupe 2,2-diméthyl-1,3-dioxolane et B est un groupement acétyl, de formule

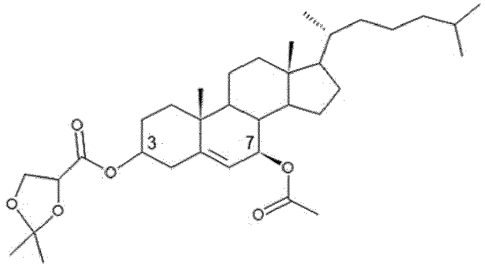

10. Composition selon l'une quelconque des revendications 1 à 9, dans laquelle ledit véhicule lipidique est une huile végétale ou un mélange d'huiles végétales.

11. Composition selon l'une quelconque des revendications 1 à 10, dans laquelle l'huile végétale est choisie parmi l'huile d'argan, l'huile d'avocat, l'huile de lin, l'huile de tournesol, de palme, de palmiste, de noix de coco, de pépins de raisins, de moutarde noire, d'oeillette, de beurre de karité, d'amande douce, de soja, d'arachide, de coton, de sésame, d'olive, de maïs, de cacao, de ricin, de Moringa (ou huile de Ben), de colza, de rocouyer, de germe de blé, de carthame, de noix, de noisette, de navette ou leurs mélanges.

12. Composition selon l'une quelconque des revendications 1 à 11, dans laquelle ledit véhicule lipidique est l'huile d'argan.

13. Composition selon l'une quelconque des revendications 1 à 12 dans laquelle la teneur en composé de formule (I) est de 0,1 à 3,5% (p/v) du volume total de la composition.

14. Composition selon l'une quelconque des revendications 1 à 13 **caractérisée en ce que** la teneur en composé de formule (I) est de 1 à 35 mg/ml, de préférence 30 mg/ml.

15. Composition selon l'une quelconque des revendications 1 à 14 **caractérisée en ce que** la teneur en véhicule lipidique est de 90 à 99% (v/:v) du volume total de la composition.

16. Composition pharmaceutique comprenant une composition selon l'une quelconque des revendications 1 à 15.

17. Composition pharmaceutique selon la revendication 16, **caractérisée en ce qu'**elle se présente sous une forme adaptée pour une administration par voie orale.

18. Procédé de préparation d'une composition selon l'une quelconque des revendications 1 à 15, comprenant les étapes suivantes :
- la préparation d'un mélange contenant le dérivé de 7beta-hydroxycholestérol de formule(I) tel que défini dans l'une quelconque des revendications 1 à 9, un véhicule lipidique et un co-solvant,
- l'ajout éventuel d'un agent anti-oxydant et
- le cas échéant, l'évaporation du co-solvant.

19. Composition selon l'une quelconque des revendications 1 à 17, pour utilisation dans le traitement d'une pathologie néoplasique ou d'une hémopathie maligne.

20. Composition pour utilisation selon la revendication 19, dans laquelle la pathologie néoplasique est le glioblastome multiforme.
